# EUROPÄISCHE PATENTSCHRIFT

(11) **EP 1 656 113 B1**
(45) Veröffentlichungstag und Bekanntmachung des Hinweises auf die Patenterteilung: **19.09.2007**
(21) Anmeldenummer: 04763906.7
(22) Anmeldetag: 07.08.2004
(51) Int. Cl.: A61K 9/00

(54) **VERFAHREN ZUR HERSTELLUNG VON BRAUSEGRANULATEN IM VAKUUM**
METHOD FOR THE PRODUCTION OF EFFERVESCENT GRANULES IN A VACUUM
PROCEDE DE PRODUCTION DE GRANULES EFFERVESCENTS SOUS VIDE

(30) Priorität: 20.08.2003 AT 20031309
(43) Veröffentlichungstag der Anmeldung: 17.05.2006
(73) Patentinhaber: Dr. Gergely & Co., 1053 Wien (AT)
(72) Erfinder: GERGELY, Irmgard, A-1053 Wien (AT); GERGELY, Thomas, A-1053 Wien (AT); GERGELY, Stefan, A-1053 Wien (AT)
(74) Vertreter: Bogensberger, Burkhard
(86) Internationale Anmeldenummer: PCT/EP2004/008879
(87) Internationale Veröffentlichungsnummer: WO 2005/018602

(56) Entgegenhaltungen:
- EP-A- 0 037 675
- EP-A- 0 076 340
- US-A- 4 678 661

## Beschreibung

### Technisches Gebiet

Die Erfindung betrifft ein Verfahren zur Herstellung von Brausegranulaten, bei dem die Reaktionspartner in einem evakuierbaren Behälter unter Gasentwicklung im Vakuum zur Reaktion gebracht werden.

### Hintergrund der Erfindung

Brausegranulate und -tabletten können durch Mischen von alkalischen, Kohlendioxid abspaltenden Bestandteilen, insbesondere Hydrogencarbonaten oder Carbonaten, mit vorzugsweise essbaren, organischen Säuren und anschließender Direkttablettierung, durch Granulierung der Brausekomponenten in einem Wirbelschichttrockner oder Granulierung und anschließender Trocknung auf Horden hergestellt werden. Bei der Herstellung von Brausegranulaten gewinnt jedoch die Granulierung im Vakuum immer mehr an Bedeutung.

Die Vakuumgranulation ist bei der so genannten "reaktiven" Granulation für Brausegranulate von Vorteil, bei der die Säuren und/oder deren saure Salze mit den alkalischen Bestandteilen zur Reaktion gebracht und granuliert werden. Die reaktiven Bestandteile reagieren miteinander nach Zugabe von polaren Lösungsmitteln wie Wasser oder Mischungen von Wasser mit Alkoholen bzw. Bindemittellösungen oder aufgrund des Hydratwassers zumindest eines reaktiven Bestandteils. Die Kontrolle dieser Reaktion erfordert allerdings besondere Maßnahmen zur Prozesssteuerung.

In der PCT/US94/02870 wird ein Verfahren zur Granulation von Brausekomponenten beschrieben, wobei das Granulat nach der Reaktion unter Vakuum getrocknet wird. Die Granulation erfolgt bei atmosphärischem Druck und schrittweiser Zugabe der Flüssigkeit, wodurch die Reaktion besser kontrolliert werden kann. Durch Anlegen eines Vakuums und Temperaturerhöhung wird die Reaktion - vor der vollständigen Umsetzung der Säure - zwischen den Brausekomponenten gestoppt und die überschüssige Flüssigkeit entfernt. Die Angaben sind allerdings unspezifisch und geben keinen Hinweis auf die Bestimmung des Endpunktes der Reaktion durch Messen der Kohlendioxid-Entwicklung.

Die US-A-4,824,664 beschreibt ein Verfahren, bei dem die zur Reaktion benötigte Flüssigkeit bei einem Vakuum von 600 mbar eingesaugt wird. Die Reaktion findet dann unter Atmosphärendruck über einen Zeitraum von 25 bis 40 Minuten statt, worauf die Reaktion durch Trocknen im Vakuum gestoppt wird. Dies ist für ein rationelles Herstellungsverfahren einerseits viel zu langsam und andererseits läuft in dieser Zeitspanne die Reaktion unkontrollierbar ab.

Die US-A-4,678,661 beschreibt ein Verfahren, bei dem die Reaktion vermeintlich durch Druckdifferenzmessung kontrolliert wird, was jedoch ohne Kontrolle des Zeitfaktors unzureichend ist. Nach jeweiliger Zwischentrocknung werden Rohstoffe zugegeben, und die Reaktion wird durch Zugabe von Flüssigkeit neuerlich gestartet, was die Produktionszeit in unerwünschter Weise verlängert.

Die EP-B1-76 340 beschreibt ein Granulationsverfahren eines pulverförmigen oder körnigen Gemisches in einem geschlossenen System unter Vakuum, wobei ein Druck über dem resultierenden Partialdruck des Lösungsmittels und unterhalb des Normaldrucks eingehalten wird. Das Vakuum bei Reaktionsstart muss möglichst niedrig sein (ca. 10 bis 20 mbar). Während der Vakuumbehandlung wird zur Passivierung der Oberfläche von zumindest einer der Reaktionskomponenten eine dosierte Menge des Lösungsmittels dem Gemisch hinzugefügt. Nach Erreichen von 1000 mbar durch die Kohlendioxid-Entwicklung ab dem Lösungsmittelzusatz, wird das Gemisch getrocknet. Diese Behandlung - Lösungsmittelzusatz und Trocknen - wird wiederholt bis die durch deutliche Verlangsamung der Reaktion bzw. verminderte Gasentwicklung angezeigte Oberflächenpassivierung erreicht ist. Die entwickelte Kohlendioxid-Menge bei 1000 mbar dient als Parameter für den Grad der Passivierung der Oberfläche. Der Verlauf der Reaktion zwischen 10 und 1000 mbar kann durch das bei der Reaktion entstehende und nicht abgesaugte Wasser bereits leicht zu einer Überreaktion und unerwünscht starker Granulierung führen.

In der US-A-4,911,930 wird ein Heißluft- bzw. Dampfstrom mittels Unterdruck in das Granulat eingesaugt und kann nicht zur Kontrolle der Reaktion dienen.

Nachteile der angeführten Verfahren sind, dass die für die Durchführung einer optimalen Reaktion notwendigen Parameter weder eindeutig von Charge zu Charge reproduzierbar, noch unabhängig vom Einfluss der chargenbedingten Rohstoffunterschiede definierbar sind. Die Reaktion der Brausekomponenten wird auch durch das während der Reaktion gebildete Wasser beeinflusst. Abhängig von der Rohstoffqualität kann die Reaktion schwächer oder stärker verlaufen, wodurch unterschiedliche Mengen Wasser pro Zeiteinheit entstehen. Aufgrund dieser variierenden Reaktionsgegebenheiten wird die Steuerung des Verfahrens alleine über die Zeit oder alleine über die Kohlendioxid-Messung maßgeblich erschwert bzw. die hoch erwünschte Möglichkeit der Automatisierung praktisch ausgeschlossen.

Wird als wesentlicher Parameter nur die Zeitspanne (wie z.B. gemäß der US-A-5,312,626 bzw. der EP-A1-525,388), in der die Reaktion abläuft, angesehen, kann diese bei unterschiedlichen Rohstoffqualitäten, z.B. bei unterschiedlicher Restfeuchte, Korngröße etc. der Säuren und/oder deren sauren Salzen oder der Kohlendioxid abspaltenden Alkalibestandteile variieren und zu unterschiedlichen Ergebnissen wie zu einer Übergranulation mit Agglomeratbildung oder zu einer nicht ausreichenden Granulation führen.

Es hat sich daher gezeigt, dass die Verfahren entsprechend dem Stand der Technik nicht geeignet waren, um standardisierte Verfahren für die vollautomatische Herstellung zu erreichen. Außerdem verlängert - wie bereits erwähnt - die Zwischentrocknung und die Wiederholung des Granulierschrittes die Produktionszeiten in unerwünschtem Maße.

### Beschreibung der Erfindung

Aufgabe der Erfindung war es, ein Verfahren zur Herstellung von Brausegranulaten zu entwickeln, das einen kontrollierten Ablauf der chemischen Reaktion und eine standardisierte, reproduzierbare Prozesssteuerung ermöglicht sowie Abweichungen der Rohstoffqualitäten kompensieren kann, beispielsweise im Hinblick auf einen vollautomatisierten computergesteuerten Ablauf. Die Aufgabe wird durch die kennzeichnenden Merkmale des Patentanspruches 1, besonders vorteilhaft aber unter Einbezug der Merkmale eines oder mehrerer abhängiger Patentansprüche gelöst, in denen zweckmäßige Aus- und Weiterbildungen der Erfindung beschrieben sind.

Erfindungsgemäß wird die gute Steuerbarkeit der Reaktion dadurch gegeben, dass die Reaktion in einem Vakuumbereich von 200 bis 900 mbar durchgeführt und die Evakuierung des Behälters auf den ersten Vakuumwert nach erfolgter Gasentwicklung auf einen zweiten Vakuumwert wiederholt, gegebenenfalls mehrfach wiederholt, wird und die ohne Zwischentrocknung in Zyklen ablaufende Reaktion anschließend durch Trocknen des entstandenen Brausegranulats unter Vakuum gestoppt wird.
Weiters durch die Wahl des ersten und zweiten Vakuumwertes und damit der Druckdifferenz für die Gasentwicklung durch die Reaktion sowie durch die Wahl der maximalen Anzahl der Zyklen, der maximalen Reaktionsdauer und gegebenenfalls - als Sicherheitsmassnahme - einer Obergrenze von z.B. 160 Ampere für die Stromaufnahme des Rührwerkes (Rührerlast).

Damit können für unterschiedliche Brausegranulate je nach Inhaltsstoffen ganz spezifische Reaktionscharakteristika erstellt werden, nach deren Vorgabe die weiteren Produktionschargen automatisch ablaufen können.

Auch bei rohstoffbedingten Unterschieden und damit verbundener Abweichung des Reaktionsverhaltens kann ein optimaler Reaktionsverlauf dadurch erreicht werden, dass nach Erreichen eines der festgelegten Maxima, d.h. der maximalen Zyklenanzahl oder der maximalen Reaktionsdauer, die Reaktion durch VakuumTrocknen abgebrochen wird.
Erfindungsgemäß werden die reaktiven Bestandteile in einem evakuierbaren Behälter miteinander unter Vakuum zur Reaktion gebracht, wobei der Behälter bis zu einem ersten Vakuumwert evakuiert wird und der erste Vakuumwert so gewählt wird, dass die Reaktion fortläuft und nicht abgebrochen wird und man dann den Druck im Behälter aufgrund der bei der Reaktion entstehenden Gase mit einer vorgegebenen Druckdifferenz bis zu einem zweiten Vakuumwert ansteigen lässt. Dieser Schritt wird zyklisch durch wiederholtes, gesteuertes Öffnen und Schließen des Ventils zur Vakuumpumpe, mit einer festgelegten Anzahl von Zyklen in einer vorgegebenen Zeit wiederholt, wonach durch Vakuumtrocknen die Reaktion gestoppt wird. Dadurch kann die Kohlendioxid- und Wasserdampfentwicklung verlangsamt und kontrolliert gesteuert werden. Für diesen Vorgang wurde der Begriff "Pendelvakuum" geprägt.

Die Kenndaten und Parameter des Pendelvakuums, wie die Druckdifferenz, der erste und zweite Vakuumwert, sowie die Anzahl der Zyklen und die Zeitspanne, in der die Zyklen ablaufen, gegebenenfalls auch das Maximum der Rührerlast, können festgelegt werden. Mit der Vorgabe dieser für den Reaktionsablauf wesentlichen Parameter, unabhängig von unterschiedlichen Rohstoffqualitäten, können alle weiteren Produktionschargen eines Produktes voll automatisch ablaufen und für jedes Produkt können diese Daten produktspezifisch festgelegt und für die weitere Produktion vorgegeben werden. Dies ist besonders wichtig für einen automatisierten, computergesteuerten Betrieb.

Ein Vorteil des erfindungsgemäßen Verfahrens ist, dass im Zuge der reaktiven Granulierung durch die Wahl des Vakuumbereiches und der gewählten Druckdifferenz sowie durch die Anzahl der Zyklen in einer im Voraus festgelegten Zeit im reduzierten Vakuum das bei der Reaktion - abhängig vom Dampfdruck bei den gewählten Vakuumwerten - entstehende Wasser oder das eingebrachte Lösungsmittel verdampft und so nicht sekundär die Reaktion beeinflusst. Dadurch werden gezielte und gut steuerbare Reaktionen ermöglicht und eine unkontrollierbare Kettenreaktion vermieden.

Durch die verlangsamte und kontrollierte Reaktion mit Pendelvakuum kann ohne Zwischentrocknung eine unmittelbare Abfolge der Reaktionszyklen stattfinden, worauf nach Abschluss der festgelegten Anzahl der Zyklen, innerhalb einer vorgegebenen Zeitspanne, das Granulat getrocknet und auf die gewünschte Korngröße zerkleinert werden kann.

Unter "Vakuum" wird in der vorliegenden Anmeldung ein Raumzustand mit einem gegenüber der umgebenden Luft verringerten Druck verstanden. Es ist wichtig, dass der Druckanstieg auf den zweiten Vakuumwert nicht bis zu dem Atmosphärendruck erfolgt, der am Standort vorherrscht. Der zweite Vakuumwert soll mindestens 10 % unter dem jeweils - am Standort - herrschenden Umgebungsdruck liegen. Die nachfolgenden Beispiele für Vakuumwerte beziehen sich auf einen Umgebungsdruck von 1 bar.

Die Druckdifferenz zwischen dem ersten und zweiten Vakuumwert soll 200 bis 700 mbar, bevorzugt 300 bis 500 mbar, betragen und die kontrollierte Reaktion zyklisch in einem Vakuumbereich von 200 bis 900 mbar ablaufen.

Der erste Vakuumwert wird so gewählt, dass ein Teil der zum Starten der Reaktion erforderlichen Flüssigkeitsmenge nach dem erstmaligen Evakuieren auf den ersten Vakuumwert im Reaktionsbehälter zurückbleibt und so für das Fortlaufen der Reaktion nach erneutem Evakuieren auf den ersten Vakuumwert ausreichend Feuchtigkeit vorhanden ist. Der Druckanstieg bis zum zweiten Vakuumwert wird in Abhängigkeit von der Reaktivität der reaktiven Bestandteile und der durch die Reaktion entstehenden Kohlendioxid- und Wasserdampfmenge festgelegt. Zur Feinsteuerung des Reaktionsverlaufes können die Verfahrensparameter, d.h. der erste und der zweite Vakuumwert und auch die Druckdifferenz, von Zyklus zu Zyklus variiert werden.

Die in Zyklen ablaufende Reaktion kann auch nach dem zusätzlichen Einbringen von Feststoffen oder Flüssigkeiten ohne Zwischentrocknung wiederholt werden.

Zur Durchführung des automatisierten Verfahrens wird der evakuierbare Behälter, beispielsweise eine Trommel oder ein Kessel, mit den die reaktiven Bestandteile enthaltenen Ausgangsstoffen beladen, die zum Reaktionsstart erforderliche Flüssigkeitsmenge zugefügt und das Programm gestartet, das beispielsweise nach den vorgegebenen Werten der Parameter: erster Vakuumwert von 500 mbar, zweiter Vakuumwert von 800 mbar, Druckdifferenz von 300 mbar, maximale Zyklenanzahl von 4 in einer maximalen Reaktionsdauer von 5 min, automatisch gesteuert ablaufen kann. Die Reaktion wird nach dem Erreichen des ersten Maximums, d.h. entweder der Anzahl der Zyklen oder der Verfahrensdauer, abgebrochen. Der Abbruch der Reaktion kann durch Vakuumtrocknung erfolgen. Danach werden die weiteren Prozessschritte, z.B. Zumischung weiterer Inhaltsstoffe, weitere Granulierung, Endtrocknung, Zerkleinerung, Sieben und Entleerung, angesteuert.

Es können verschiedene Arten von Vakuumpumpen eingesetzt werden, wie Drehschieber-, Flüssigkeitsring- oder Schraubenrotorpumpen, mit einem an die Behältergröße angepassten Nennsaugvermögen, die in der Lage sein sollen, einen Enddruck von 0,1 mbar zu erreichen und den leeren Behälter in 30 sec bis 2 min auf 10 mbar zu evakuieren.

Bei einer reaktiven Granulation kann das erfindungsgemäße Verfahren, unabhängig von der Temperatur und Art, wie die Reaktion gestartet wird, Anwendung finden. Die Temperatur, bei der das erfindungsgemäße Verfahren durchgeführt wird, ist nicht kritisch. Es kann bei Raumtemperatur (20°C) oder bei einer erhöhten Produkttemperatur von z.B. 40 bis 80 °C gearbeitet werden. Die Flüssigkeit, die als Granulierflüssigkeit dient, kann entweder auf einen der Reaktionspartner, wie die essbaren organischen Säuren oder die Kohlendioxid abspaltenden alkalischen Brausebestandteile, aufgebracht werden, bevor der zweite Reaktionspartner zugefügt wird, oder direkt zu einer Mischung der Brausekomponenten eingebracht werden. Das Einbringen der Flüssigkeit kann, wie in der US 4 824 664 beschrieben, durch Einsaugen unter Vakuum erfolgen. Hat der Rohstoff eines oder beider Reaktionspartner einen höheren Anteil an Restfeuchte, so laufen die Zyklen schneller ab, wobei entsprechend des erfindungsgemäßen Verfahrens, das nicht nur zeitgesteuert ist, durch die vorgegebene Anzahl der Zyklen eine Überreaktion und Übergranulation verhindert wird. Bei geringerer Restfeuchte laufen die Zyklen verlangsamt ab, es wird aber in diesem Falle durch die maximal festgelegte Verfahrensdauer trotzdem die erforderliche Reaktion und Granulierung erzielt.

Als Flüssigkeiten können für Brausegranulate, abgesehen von polaren Lösungsmitteln, auch Bindemittellösungen in Wasser, Alkoholen oder Mischungen davon eingesetzt werden, wie z.B. Polyvinylpyrrolidone, Polyethylenglycol bzw. Hydroxypropylmethylcellulose, Zuckerlösungen oder Lösungen von Zuckeralkoholen bzw. Kolloiden. Weiters können reaktive Lösungen eingesetzt werden, wie z.B. Lösungen von organischen Säuren in Wasser oder Wasser/Ethanol, oder von sauren Salzen der essbaren organischen Säuren oder von deren alkalischen Salzen.

Zu den reaktiven Bestandteilen zählen bei Brausegranulaten zumindest eine saure Brausekomponente, d.h. eine feste, organische Säure und/oder deren Salze, und zumindest eine Kohlendioxid M abspaltende, alkalische Brausekomponente. Die organische Säure ist bevorzugt essbar. Es können auch mehrere verschiedene organische Säuren und/oder deren Salze und/oder Kohlendioxid abspaltende Brausekomponenten miteinander zur Reaktion gebracht werden. Weiters können in bestimmten Ausführungsformen der Erfindung andere Komponenten, beispielsweise Magnesiumoxyd, als reaktive Bestandteile enthalten sein.

Das erfindungsgemäße Verfahren ist weiters geeignet für die Herstellung von Brausegranulaten, bei der die Freisetzung von Wasser aus Hydraten der reaktiven Bestandteile unter Temperatur zur Granulierung genützt wird. Als "Hydrat" werden chemische Verbindungen von organischen oder anorganischen Substanzen mit H2O verstanden, wobei das H2O nicht Bestandteil von Komplexverbindungen ist. Das gebundene H2O wird auch als Kristallwasser oder Hydratwasser bezeichnet.

Hierfür können wasserhaltige organische Säuren wie z.B. ZitronensäureMonohydrat oder wasserhaltiges Natriumcarbonat verwendet werden, die bei steigender Temperatur Wasser abgeben, das für die reaktive Granulierung erforderlich ist. Dieser Prozess ist bekannt als "schwer kontrollierbar, um reproduzierbare Ergebnisse zu erzielen" (Lachman & Lieberman: Pharmaceutical dosage forms, 1980; Seite 233). Mit dem erfindungsgemäßen Verfahren kann hingegen ein gut kontrollierbarer und reproduzierbarer Prozess durchgeführt werden, bei dem eine Anzahl von bis zu 100 Zyklen, gegebenenfalls sogar mehr als 100 Zyklen, des Pendelvakuums zwischen zwei festgelegten Vakuumwerten in einer bestimmten Zeit oder bis zur Erwärmung der Masse auf eine Temperatur von 30 bis 80°C abläuft, wodurch ein Teil des Wassers (die Menge ist abhängig vom Dampfdruck des Wassers bei der gewählten Temperatur und dem gewählten Vakuumwert) sowie ein Teil des Kohlendioxids bei den sich wiederholenden Zyklen abgesaugt wird und den Prozess nicht mehr unkontrolliert beeinflussen kann.

Das erfindungsgemäße Verfahren kann für die Herstellung der verschiedensten Brausegranulate und von Brausetabletten, die aus diesen Brausegranulaten herstellbar sind, verwendet werden, z.B.:
- Granulate mit pharmazeutischen Wirkstoffen, die mit den sauren Brausekomponenten oder den alkalischen Brausekomponenten reagieren.
- Granulate mit pharmazeutischen Wirkstoffen, die mit den eingesetzten Brausekomponenten nicht reagieren, aber gemeinsam mit der Brausebasis granuliert werden,
- Basisbrausegranulate, die nach der Granulation mit für Brausetabletten geeigneten pharmazeutischen Wirkstoffen und gegebenenfalls Hilfsstoffen, Neutralstoffen und Aromen vermischt werden. Beispiele für geeignete Gruppen von Wirkstoffen sind: Analgetika, Antipyretika, Antihistaminika, Antiallergika, Antibiotika, Antidiabetika, Onkolytika, Expektorantien, Elektrolytpräparate, Laxantien, Vitamine, Phytopharmaka, Herz/Kreislaufmittel, Antidiarrhoemittel, Diuretika und durchblutungsfördernde Mittel.

In einer weiteren Ausführungsform wurde nun heraus gefunden, dass durch eine zusätzliche, nicht reaktionsbedingte Erhöhung des Kohlendioxid-Partialdrucks im Reaktionsbehälter zumindest ein Teil der an den Brausekristallen nach Vakuumtrocknung immer noch anhaftenden Restfeuchte "inaktiviert" und somit das Brausesystem lagerstabiler gemacht werden kann. Üblicherweise liegt der Restfeuchtegehalt, je nach Brausesystem, in einem Bereich von 0,01 bis 1 Gew.%, insbesondere in einem Bereich von ca. 0,1 bis 0,8 Gew.%.

Bei besonders reaktiven Systemen erwies sich die zusätzliche Einleitung von Kohlendioxid als vorteilhaft, um den Prozess der reaktiven Granulierung noch besser steuerbar zu machen. Überraschenderweise zeigte sich dabei, dass dies gleichzeitig zu einer Stabilisierung des Granulates im Sinne einer verminderten Empfindlichkeit gegenüber der verbliebenen Restfeuchte führte, was sich mit speziellen eigenen Messgeräten anhand der Kohlendioxid-Freisetzung aus dem fertigen Produkt überprüfen ließ. Diese Erkenntnis wird in einer weiteren Ausführungsform des erfindungsgemäßen Verfahrens durch die zusätzliche Einleitung von Kohlendioxid im Pendelprozess und/oder bei der anschließenden Endtrocknung genutzt.

Der erwähnte vorteilhafte Effekt wird dadurch erreicht, dass man nach Anlegen eines Vakuums im Zuge der Reaktionsgranulierung von Brausesystemen, wie beispielsweise bei der erfindungsgemäßen zyklischen Reaktionsgranulierung unter Pendelvakuum, vor allem aber im Verlauf der Endtrocknung von solcherart hergestellten Brausesystemen, zusätzliches Kohlensäuregas aus einer externen Quelle in den Reaktionsbehälter unter Rühren einströmen lässt. Auf diese Weise kann bei der Reaktionsgranulierung während des Ablaufs der Zyklen und beim Endtrocknen der Systeme der erhöhte Kohlendioxid-Partialdruck zu einer weiteren Reduktion der Reaktion führen, sodass - bedingt durch das Einströmen des Kohlendioxids bei der Reaktionsgranulierung - die Zyklenanzahl typischerweise zu erhöhen ist und gegebenenfalls bis zu zehnmal mehr Zyklen ablaufen als bei Reaktionsführung ohne externe Kohlendioxidzufuhr.

Durch speziell dafür entwickelte Messgeräte aus eigenem Hause, mit deren Hilfe winzigste Gasmengen in der Größenordnung von Mikrolitern exakt gemessen und dokumentiert werden können, gelingt es, Brausesysteme unabhängig von der Art ihrer Herstellung auf ihre Reaktivität durch die Restfeuchte zu analysieren. Anhand solcher Messungen kann der Nachweis geführt werden, dass die Anwendung der zusätzlichen Kohlendioxid-Partialdruckerhöhung tatsächlich zu einer signifikant verbesserten Stabilität der Brausesysteme führt.

In einer weiteren Ausführungsform wird - entweder zusätzlich oder erstmalig - nach Beendigung der Reaktionsgranulierung durch wiederholte Implosion von Kohlendioxidgas in den Reaktionsbehälter der im Behälter vorherrschende Kohlendioxid-Partialdruck erhöht. Durch diese Maßnahme gelingt es, die Brausepartikel so stark mit Kohlendioxid zu umhüllen bzw. zu sättigen, dass selbst bei längerer Lagerung des Brausegranulates offenbar eine Kohlendioxid-Mikroatmosphäre bestehen bleibt, die eine Weiterreaktion der sauren und alkalischen Komponenten miteinander wirkungsvoll hemmt oder unterbindet.

Es ist bekannt, dass zahlreiche pharmazeutische Wirkstoffe wie z.B. Acetylsalicylsäure oder Acetylcystein sehr empfindlich auf Restfeuchtegehalte in Brauseformulierungen reagieren, weil beispielsweise bei Acetylsalicylsäure durch Verseifung freie Essigsäure entsteht, die ihrerseits wieder eine sekundäre Kettenreaktion auslösen kann. Gerade solche Sekundärreaktionen können nun aber dank der erfindungsgemäßen Maßnahme der Stabilitätsverbesserung durch Kohlendioxid-Partialdruckerhöhung deutlich reduziert werden. Es ist ein weiterer Vorteil dieser Maßnahme, dass sie nicht nur auf eine spezielle Art der Brauseherstellung, wie beispielsweise die Reaktionsgranulierung nach dem erfindungsgemäßen Pendelvakuumverfahren, sondern ganz allgemein auf beliebige partikuläre Brausesysteme wie Brausepulver und Brausegranulate, unabhängig von der Art ihrer Herstellung, anwendbar ist.

Die Erfindung wird durch die nachfolgenden Beispiele weiter erläutert.

### Beispiel 1: Reaktive Granulierung ohne Zusatz von Granulierflüssigkeit

In einen beheizbaren Vakuumgranulator wird wasserfreies Natriumhydrogencarbonat und Zitronensäuremonohydrat in einem dem gewünschten pH-Wert entsprechenden Verhältnis beladen und 5 min bis zur Homogenität vermischt.

Bei ansteigender Temperatur wird die Reaktion durch das aus dem Zitronensäuremonohydrat freigesetzte Wasser gestartet. Für die Reaktion wird ein Pendelvakuum mit 2 vorgewählten Vakuumwerten, z.B. 550 und 900 mbar, gewählt, wobei auf 550 mbar evakuiert wird und das Ventil zur Vakuumpumpe geschlossen wird. Durch die Reaktion kommt es zu einem Druckanstieg bis 900 mbar. Bei diesem Wert wird das Ventil wieder geöffnet, der Kessel erneut auf 550 mbar evakuiert und dieser Vorgang mehrfach wiederholt. Nach einer Reaktionsdauer von 20 bis 40 min oder nach Erreichen einer Temperatur von 40 bis 60 °C wird das Pendelvakuum weggeschaltet und das Granulat mit voller Pumpenleistung vakuumgetrocknet.

### Beispiel 2: Reaktive Granulierung mit Zusatz von Granulierflüssigkeit (Wasser)

Herstellung eines Brausegranulates, das für verschiedenste pharmazeutische Wirkstoffe und/oder Wirkstoffkombinationen, u.a. Vitamine und Spurenelemente, eingesetzt werden kann, wobei das auf die gewünschte Korngröße zerkleinerte Brausegranulat mit den entsprechenden Wirkstoffen sowie Süßstoffen und gegebenenfalls Aromen sowie Füllstoffen gemischt wird. Das Granulat kann entweder in Sachets abgefüllt werden oder bei Bedarf mit Schmiermittel versetzt und zu Tabletten verpresst werden.

Ein Vakuumgranulator mit beheizbarem Mantel wird beladen mit 31,76 Gew. Teilen Zitronensäure, die unter Rühren auf 50 ° C aufgeheizt wird. Bei Erreichen der Temperatur werden 0,16 Gew. Teile Wasser unter Rühren zugefügt und 5 min verteilt. Anschließend werden 12,3 Gew. Teile Natriumhydrogencarbonat zugegeben, der Rührer und das Pendelvakuum für die Reaktionssteuerung eingeschaltet mit dem vorgegebenen ersten Vakuumwert = 450 mbar, zweiten Vakuumwert = 850 mbar und der Anzahl von 4 Zyklen ("Pendel") innerhalb von maximal 4 min.
Nach Ende des 4. Zyklus (Pendels), z.B. nach 3½ min, spätestens jedoch nach Ablauf von 4 min und unabhängig davon, ob in dieser Zeit tatsächlich 4 Zyklen erreicht wurden, wird das Programm abgeschaltet und volles Vakuum zum Trocknen des Granulates angelegt. Das getrocknete Granulat wird auf die gewünschte Korngröße gesiebt und kann je nach Bedarf als Basisbrausegranulat verwendet werden.

Für den vollautomatischen Betrieb können die ermittelten Kenndaten für das Produkt, d.h. Vakuumbereich, erster und zweiter Vakuumwert, Druckdifferenz, Anzahl der Zyklen und Dauer des Pendelvakuums, eingestellt werden, wodurch das Verfahren schrittweise nach dem jeweiligen Erreichen der eingestellten Werte ablaufen kann.

### Beispiel 3 Magnesiumbrausegranulat

In einem Vakuumgranulator mit beheizbarem Mantel werden eingebracht: 31,4 Gew. Teile Zitronensäure, 5,9 Gew. Teile Magnesiumcarbonat sowie gegebenenfalls Süßstoffe. Unter Rühren wird auf 50 °C aufgeheizt. Anschließend werden 0,9 Gew. Teile Wasser unter Rühren zugegeben und das Programm eingeschaltet. Die Reaktion verläuft mit Pendelvakuum bei den vorgegebenen Werten zwischen 500 und 900 mbar mit 5 Zyklen in maximal 9 min ab.

Je nach Reaktivität der Säure und des Carbonats wird entweder nach dem 5. Zyklus oder nach der maximalen vorgegebenen Zeit von 9 min, je nachdem, welches der beiden festgelegten Maxima zuerst erreicht wird, das Pendelvakuum abgeschaltet.

Danach werden 4,4 Gew. Teile Kaliumhydrogencarbonat, 3,0 Gew. Teile Magnesiumoxyd sowie 1,0 Gew. Teile Zitronensäure zugemischt und die Mischung wird mit 0,55 Gew. Teilen einer Zitronenlösung in 50 %igem Ethanol unter Rühren versetzt. Die Reaktion verläuft unter einem zweiten, vorgegebenen Pendelvakuum zwischen 450 und 750 mbar mit 2 Zyklen in maximal 5 min. Nach dem 2. Zyklus oder nach 5 min wird das Pendelvakuum abgeschaltet, und das Produkt unter langsamem Rühren mit Voll-Vakuum getrocknet. Nach dem Sieben auf die gewünschte Körnung kann zu dem erhaltenen Granulat Aroma zugemischt werden und das Granulat entweder in Sachets abgefüllt oder zu Tabletten verpresst werden.

### Beispiel 4:

Es wurde das Verfahren nach der EP-B-0 076 340 (Stand der Technik) mit dem erfindungsgemäßen Verfahren verglichen.

### a) Verfahren nach der EP-B-0 076 340 (Vergleichsversuch)

In einem Vakuumgranulator wurden Zitronensäure, Ascorbinsäure und Süßstoffe auf 50°C aufgeheizt. Dann wurde Natriumhydrogencarbonat zugemischt und auf 10 mbar evakuiert. Nun wurden 21 ml Wasser zugegeben und die Reaktion gestartet. Der Druck stieg in 30 sec auf 1 bar an, wobei das Granulat sehr plastisch wurde und am Rührer anklebte, wodurch es fast zu einer Blockade des Rührers kam.

Danach wurde das Produkt mittels Vakuums bis auf 20 mbar in 15 min getrocknet. Nach einer weiteren Zugabe von 21 ml Wasser wurde die Reaktion erneut gestartet und der Druck stieg in 45 sec auf 1 bar an, wobei das Granulat sehr plastisch wurde und sich teils große kugelförmige Agglomerate bildeten. Es erfolgte eine Zugabe von Natriumcarbonat und anschließende Trocknung, wobei das Produkt nur langsam trocknete und in 25 min nur 17 mbar erreicht werden konnten.

### b) Verfahren nach der Erfindung

Im gleichen Vakuumgranulator wurden Zitronensäure, Ascorbinsäure und Süßstoffe auf 50°C aufgeheizt. Danach wurden Natriumhydrogencarbonat zugemischt und 21 ml Wasser zugegeben. Nun wurde ein Pendelvakuum eingeschaltet, festgelegt zwischen einem ersten Vakuumwert von 500 mbar und einem zweiten Vakuumwert von 900 mbar. Es wurden 3 Zyklen in 65 sec durchgeführt. Die Masse war leicht schollig, nur etwas plastisch und konnte vom Rührer gut durchgemischt werden, ohne dass es zu einer Blockierung oder Knollenbildung kam. Danach erfolgte die Zugabe von Natriumcarbonat und anschließende Trocknung, bei der 15 mbar in 17 min erreicht wurden.

### Ergebnis:

Das erfindungsgemäße Verfahren ist wesentlich kürzer, und die Granulation erfolgt deutlich kontrollierter und gleichförmiger (eine Überreaktion wird verhindert). Nach dem Verfahren der EP-B-0 076 340 ist ein zusätzlicher Verfahrensschritt mit Trocknung, nochmaliger Flüssigkeitszugabe und erneuter vollständiger Durchführung der Reaktion notwendig, um ein dem erfindungsgemäßen Verfahren gleichwertiges, d.h. stabiles, Produkt zu erhalten. Durch den zusätzlichen Verfahrensschritt einer zweiten Granulierung mit Trocknung dauert das Verfahren nach dem Stand der Technik wesentlich länger und die kritisch ablaufende Granulationsreaktion muss ein zweites Mal durchgeführt werden, wobei durch die Bildung von teils großen kugelförmigen Agglomeraten eine uneinheitliche Granulatstruktur entsteht.

### Beispiel 5 Kohlendioxid-Partialdruckerhöhung

Dieses Beispiel wurde entsprechend Beispiel 4 b) durchgeführt, jedoch mit Kohlendioxid-Partialdruckerhöhung, wie nachfolgend beschrieben.

In einem Vakuumgranulator wurden Zitronensäure, Ascorbinsäure, Süßstoffe und Natriumhydrogencarbonat so lange mit Pendelvakuum unter Einsaugen von Kohlendioxid während der Zyklen aufgeheizt, bis 50° C erreicht wurden, wobei bei jedem Zyklus auf 200 mbar evakuiert wurde und anschließend ein Druckanstieg auf 800 mbar erfolgte. Nach Zugabe von 21 ml Wasser wurden weitere 10 Zyklen unter Einströmen von Kohlendioxid durchgeführt. Nach Zugabe von Natriumcarbonat wurde das Granulat mittels Vakuum getrocknet, wobei bei der Endtrocknung weitere 20 Zyklen unter Einströmen von Kohlendioxid durchgeführt wurden. Dieses Granulat zeigte bei der Überprüfung der Lagerstabilität nach einer Woche um 30% verbesserte Werte gegenüber der nach Beispiel 4 b) hergestellten Kontrollprobe.

## Patentansprüche

1. Verfahren zur Herstellung von Brausegranulaten, bei dem als reaktive Bestandteile zumindest eine saure Brausekomponente und zumindest eine CO2-abspaltende alkalische Brausekomponente sowie gegebenenfalls eine Granulierflüssigkeit in einen evakuierbaren Behälter geladen und unter Rühren im Vakuum miteinander zur Reaktion gebracht werden, wobei der Behälter nach Beladung mit den reaktiven Bestandteilen bis zu einem ersten Vakuumwert evakuiert wird, worauf - nach reaktionsbedingter Gasentwicklung und Druckanstieg bis zu einem zweiten Vakuumwert - das entstandene Brausegranulat unter Vakuum getrocknet wird,
**dadurch gekennzeichnet, dass** die Reaktion in einem Vakuumbereich von 200 bis 900 mbar durchgeführt und die Evakuierung des Behälters auf den ersten Vakuumwert nach erfolgter Gasentwicklung wiederholt, gegebenenfalls mehrfach wiederholt, wird und die ohne Zwischentrocknung in Zyklen ablaufende Reaktion anschließend durch Trocknen des entstandenen Brausegranulats unter Vakuum gestoppt wird.

2. **Verfahren nach Anspruch 1, dadurch gekennzeichnet, dass als erster Va**kuumwert ein Wert im Bereich von 200 bis 700 mbar, insbesondere von 300 bis 600 mbar, vorgegeben wird,

3. Verfahren nach Anspruch 1 oder 2, **dadurch gekennzeichnet, dass** als Druckdifferenz zwischen erstem und zweitem Vakuumwert ein Wert von 200 bis 700 mbar, vorzugsweise von 300 bis 500 mbar vorgegeben wird und der zweite Vakuumwert höchstens 900 mbar beträgt.

4. Verfahren nach einem der Ansprüche 1 bis 3, **dadurch gekennzeichnet, dass** der erste Vakuumwert und/oder der zweite Vakuumwert von Zyklus zu Zyklus variiert werden.

5. Verfahren nach Anspruch 3 oder 4, **dadurch gekennzeichnet, dass** die Druckdifferenz von Zyklus zu Zyklus variiert wird.

6. Verfahren nach einem der Ansprüche 1 bis 5, **dadurch gekennzeichnet, dass** für die Reaktionsgranulierung eine maximale Anzahl von Zyklen und/oder eine maximale Reaktionsdauer im Voraus festgelegt und die Reaktion nach Erreichen eines der beiden Maxima gestoppt wird.

7. Verfahren nach Anspruch 6, **dadurch gekennzeichnet, dass** eine Zyklenzahl von 2 bis 100 festgelegt wird.

8. Verfahren nach einem der Ansprüche 6 bis 7, **dadurch gekennzeichnet, dass** ein Zyklus 30 bis 240 Sec dauert.

9. Verfahren nach einem der Ansprüche 6 bis 7, **dadurch gekennzeichnet, dass** für die Reaktionsgranulierung eine Reaktionsdauer von 1 bis 40 min, insbesondere von 1 bis 15 min, festgelegt wird.

10. Verfahren nach einem der Ansprüche 1 bis 9, **dadurch gekennzeichnet, dass** die Reaktionsgranulierung bei einer Temperatur von 20 bis 80 °C, vorzugsweise von 40 bis 60 ° C, durchgeführt wird.

11. Verfahren nach einem der Ansprüche 1 bis 10, **dadurch gekennzeichnet, dass** zumindest einer der reaktiven Brausebestandteile oder die Mischung der reaktiven Brausebestandteile mit einer Granulierflüssigkeit versetzt wird, die vor oder während dem ersten Evakuierungsschritt in den Behälter eingebracht, insbesondere eingesaugt, wird.

12. Verfahren nach einem der Ansprüche 1 bis 11, **dadurch gekennzeichnet, dass** zumindest ein reaktiver Brausebestandteil als Hydrat vorliegt.

13. Verfahren nach einem der Ansprüche 1 bis 12, **dadurch gekennzeichnet, dass** als saure Brausekomponenten essbare organischen Säuren und/oder deren Salze, und als alkalische Brausekomponenten Carbonate und/oder Hydrogencarbonate und/oder Magnesiumoxid eingesetzt werden.

14. Verfahren nach einem der Ansprüche 1 bis 13, **dadurch gekennzeichnet, dass** das Brausegranulat nach dem Trocknungsschritt mit zumindest einem pharmazeutischen Wirkstoff und gegebenenfalls Hilfsstoffen, Neutralstoffen, Süßstoffen und/oder Aromen vermischt wird.

15. Verfahren nach Anspruch 14, **dadurch gekennzeichnet, dass** das Brausegranulat mit zumindest einem Wirkstoff aus der Gruppe der Analgetika, Antipyretika, Antihistaminika, Antiallergika, Antibiotika, Antidiabetika, Onkolytika, Expektorantien, Elektrolyte, Laxantien, Vitamine, Phytopharmaka, Herz/Kreislaufmittel, Antidiarrhoemittel, Diuretika und durchblutungsfördernden Mitteln vermischt wird.

16. Verfahren nach einem der Ansprüche 1 bis 15, **dadurch gekennzeichnet, dass** während der Reaktionszyklen Kohlendioxid eingeleitet wird.

17. Verfahren nach einem der Ansprüche 1 bis 16, **dadurch gekennzeichnet, dass** nach erfolgter Trocknung Kohlendioxid in den Behälter eingesaugt und das Brausegranulat, vorzugsweise unter Rühren, mit Kohlendioxid behandelt wird.

18. Verfahren zur Stabilisierung von Restfeuchtigkeit enthaltenden Brausepartikeln, **dadurch gekennzeichnet dass** die Brausepartikel im Zuge ihrer Herstellung oder danach mit Kohlendioxid behandelt werden.

19. Verfahren nach Anspruch 18, **dadurch gekennzeichnet, dass** die Behandlung der Brausepartikel in einem geschlossenen Behälter in einer Kohlendioxid-angereicherten Atmosphäre, vorzugsweise unter Rühren, erfolgt.

20. Brausepartikel erhältlich in einem Verfahren gemäss einem der Ansprüche 16 bis 19, **dadurch gekennzeichnet, dass** sie in einer mit gasförmigem Kohlendioxid angereicherten Form vorliegen.

21. Brausepartikel nach Anspruch 20, **dadurch gekennzeichnet, dass** sie einen Restfeuchtegehalt von 0,01 bis 1 Gew.%, insbesondere von 0,1 bis 0,8 Gew.%, aufweisen und vorzugsweise als Brausegranulat oder Brausepulver vorliegen.

## Claims

1. Method for the production of effervescent granules, in which at least one acidic effervescent component and at least one CO₂-eliminating alkaline effervescent component as reactive constituents and optionally a granulating liquid are loaded into an evacuatable container and are reacted with one another with stirring in a vacuum, the container being evacuated to a first vacuum value after loading with the reactive constituents, whereupon - after reaction-related gas evolution and pressure increase to a second vacuum value - the resulting effervescent granules are dried in a vacuum, **characterized in that** the reaction is carried out in a vacuum range of 200 to 900 mbar and the evacuation of the container to the first vacuum value is repeated, optionally repeated several times, after gas evolution is complete, and the reaction taking place in cycles without intermediate drying is then stopped by drying the resulting effervescent granules in a vacuum.

2. Method according to Claim 1, **characterized in that** a value in the range of 200 to 700 mbar, in particular of 300 to 600 mbar, is specified as the first vacuum value.

3. Method according to Claim 1 or 2, **characterized in that** a value of 200 to 700 mbar, preferably of 300 to 500 mbar, is specified as the pressure difference between first and second vacuum value, and the second vacuum value is not more than 900 mbar.

4. Method according to any of Claims 1 to 3, **characterized in that** the first vacuum value and/or the second vacuum value are varied from cycle to cycle.

5. Method according to Claim 3 or 4, **characterized in that** the pressure difference is varied from cycle to cycle.

6. Method according to any of Claims 1 to 5, **characterized in that** the reaction granulation specifies a maximum number of cycles and/or maximum duration of reaction in advance, and the reaction is stopped after reaching one of the two maxima.

7. Method according to Claim 6, **characterized in that** a number of cycles from 2 to 100 is specified.

8. Method according to either of Claims 6 and 7, **characterized in that** a cycle lasts for 30 to 240 sec.

9. Method according to either of Claims 6 and 7, **characterized in that** a duration of reaction of 1 to 40 min, in particular of 1 to 15 min, is specified for the reaction granulation.

10. Method according to any of Claims 1 to 9, **characterized in that** the reaction granulation is carried out at a temperature of 20 to 80°C, preferably 40 to 60°C.

11. Method according to any of Claims 1 to 10, **characterized in that** a granulating liquid is added to at least one of the reactive effervescent constituents or to the mixture of the reactive effervescent constituents and is introduced, in particular aspirated, into the container before or during the first evacuation step.

12. Method according to any of Claims 1 to 11, **characterized in that** at least one reactive effervescent constituent is present as a hydrate.

13. Method according to any of Claims 1 to 12, **characterized in that** edible organic acids and/or salts thereof are used as acidic effervescent components, and carbonates and/or bicarbonates and/or magnesium oxide are used as alkaline effervescent components.

14. Method according to any of Claims 1 to 13, **characterized in that** the effervescent granules are mixed with at least one pharmaceutical active substance and optionally excipients, neutral substances, sweeteners and/or flavours after the drying step.

15. Method according to Claim 14, **characterized in that** the effervescent granules are mixed with at least one active substance from the group consisting of the analgesics, antipyretic agents, antihistamines, antiallergic agents, antibiotics, antidiabetic agents, oncolytic agents, expectorants, electrolytes, laxatives, vitamins, phytopharmaceuticals, cardiovascular agents, antidiarrhoea agents, diuretics and agents which stimulate blood flow.

16. Method according to any of Claims 1 to 15, **characterized in that** carbon dioxide is passed in during the reaction cycles.

17. Method according to any of Claims 1 to 16, **characterized in that**, after drying is complete, carbon dioxide is aspirated into the container and the effervescent granules are treated with carbon dioxide, preferably with stirring.

18. Method for stabilizing effervescent particles containing residual moisture, **characterized in that** the effervescent particles are treated with carbon dioxide in the course of their production of thereafter.

19. Method according to Claim 18, **characterized in that** the treatment of the effervescent particles is effected in a carbon dioxide-enriched atmosphere in a closed container, preferably with stirring.

20. Effervescent particles obtainable in a method according to any of Claims 16 to 19, **characterized in that** they are present in a form enriched with gaseous carbon dioxide.

21. Effervescent particles according to Claim 20, **characterized in that** they have a residual moisture content of 0.01 to 1% by weight, in particular of 0.1 to 0.8% by weight, and are preferably present as effervescent granules or effervescent powder.

## Revendications

1. Procédé de production de granulés effervescents, dans lequel un récipient susceptible d'être mis sous vide est chargé avec - en tant qu'ingrédients réactifs - au moins un composant effervescent acide et au moins un composant effervescent alcalin susceptible de libérer du CO2, ainsi que, le cas échéant, un liquide facilitant la granulation ; les ingrédients sont ensuite amenés à réagir les uns avec les autres sous agitation et sous vide ; le récipient après le chargement avec les ingrédients réactifs est amené à un premier niveau de vide ; puis - après la formation de gaz résultant de la réaction et une augmentation de la pression jusqu'à un second niveau de vide
- les granulés effervescents obtenus sont séchés sous vide ;
**caractérisé en ce que** la réaction s'effectue dans une plage de vide allant de 200 à 900 mbars et **en ce que** la mise sous vide du récipient au premier niveau de vide est répétée après la formation de gaz résultant de la réaction, le cas échéant plusieurs fois et, ensuite, la réaction s'effectuant en cycles sans séchage intermédiaire est arrêtée par le séchage sous vide des granulés effervescents obtenus.

2. Procédé selon la revendication 1, **caractérisé en ce que** le premier niveau de vide a une valeur prédéterminée, dans la plage allant de 200 à 700 mbars et, plus particulièrement, de 300 à 600 mbars.

3. Procédé selon la revendication 1 ou la revendication 2, **caractérisé en ce que** la différence de pression entre le premier niveau de vide et le second niveau de vide a une valeur prédéterminée dans la plage allant de 200 à 700 mbars et, de préférence, de 300 à 500 mbars et **en ce que** le second niveau de vide atteint au maximum 900 mbars.

4. Procédé selon l'une des revendications 1 à 3, **caractérisé en ce que** le premier niveau de vide ou / et le second niveau de vide est / sont modifiés d'un cycle à l'autre.

5. Procédé selon la revendication 3 ou la revendication 4, **caractérisé en ce que** la différence de pression est modifiée d'un cycle à l'autre.

6. Procédé selon l'une des revendications 1 à 5, **caractérisé en ce que** l'on définit par avance pour la réaction de granulation un nombre maximum de cycles et / ou une durée de réaction maximale, et **en ce que** la réaction est arrêtée après qu'un des deux maxima est atteint.

7. Procédé selon la revendication 6, **caractérisé en ce que** le nombre de cycles est choisi dans la plage allant de 2 à 100.

8. Procédé selon l'une des revendications 6 à 7, **caractérisé en ce qu'**un cycle dure de 30 à 240 secondes.

9. Procédé selon l'une des revendications 6 à 7, **caractérisé en ce que** l'on choisit pour la réaction de granulation une durée de réaction dans la plage allant de 1 à 40 minutes et, plus particulièrement, de 1 à 15 minutes.

10. Procédé selon l'une des revendications 1 à 9, **caractérisé en ce que** la réaction de granulation est effectuée à une température dans la plage allant de 20 à 80 °C et, de préférence, de 40 à 60 °C.

11. Procédé selon l'une des revendications 1 à 10, **caractérisé en ce qu'**au moins un des ingrédients réactifs effervescents ou la mixture des ingrédients réactifs effervescents est mélangé avec un liquide facilitant la granulation, qui est introduit ou, plus particulièrement, aspiré, dans le récipient avant ou pendant la première étape de mise sous vide.

12. Procédé selon l'une des revendications 1 à 11, **caractérisé en ce qu'**au moins un des ingrédients réactifs effervescents est présent sous la forme d'un hydrate.

13. Procédé selon l'une des revendications 1 à 12, **caractérisé en ce que** l'on utilise, en tant que composants effervescents acides, des acides organiques de qualité alimentaire et / ou leurs sels et, en tant que composants effervescents alcalins, le carbonate et / ou l'hydrogénocarbonate et / ou l'oxyde de magnésium.

14. Procédé selon l'une des revendications 1 à 13, **caractérisé en ce que** les granulés effervescents sont mélangés après l'étape de séchage avec au moins un agent actif du point de vue pharmaceutique et, le cas échéant, avec des adjuvants, des substances neutres, des substances édulcorantes et / ou des agents aromatisants.

15. Procédé selon la revendication 14, **caractérisé en ce que** les granulés effervescents sont mélangés avec au moins un agent actif, choisi dans le groupe des analgésiques, des antipyrétiques, des antihistaminiques, des antiallergiques, des antibiotiques, des antidiabétiques, des oncolytiques, des expectorants, des électrolytes, des laxatifs, des vitamines, des agents phytopharmaceutiques, des médicaments pour le coeur / pour la circulation sanguine, des agents antidiarrhéiques, des agents diurétiques et des agents favorisant la circulation sanguine.

16. Procédé selon l'une des revendications 1 à 15, **caractérisé en ce que**, pendant les cycles de réaction, on introduit du dioxyde de carbone.

17. Procédé selon l'une des revendications 1 à 16, **caractérisé en ce que**, après avoir effectué le séchage, on aspire du dioxyde de carbone dans le récipient et **en ce que** les granulés effervescents sont traités par le dioxyde de carbone, de préférence, sous agitation.

18. Procédé pour la stabilisation de particules effervescentes contenant de l'humidité résiduelle, **caractérisé en ce que** les particules effervescentes sont traitées au cours de leur production ou après, avec du dioxyde de carbone.

19. Procédé selon la revendication 18, **caractérisé en ce que** le traitement des particules effervescentes se fait dans un récipient fermé sous une atmosphère enrichie en dioxyde de carbone, de préférence sous agitation.

20. Particules effervescentes pouvant être obtenues par un procédé selon l'une des revendications 16 à 19, **caractérisées en ce qu'**elles sont présentes sous une forme enrichie par du dioxyde de carbone gazeux.

21. Particules effervescentes selon la revendication 20, **caractérisées en ce qu'**elles ont une teneur en humidité résiduelle située dans la plage allant de 0,01 à 1 % en poids et, plus particulièrement, de 0,1 à 0,8 % en poids et **en ce qu'**elles se présentent, de préférence, sous la forme de granulés effervescents ou de poudre effervescente.
